# EUROPÄISCHE PATENTSCHRIFT

(11) **EP 2 034 946 B1**
(45) Veröffentlichungstag und Bekanntmachung des Hinweises auf die Patenterteilung: **27.10.2010**
(21) Anmeldenummer: 07725127.0
(22) Anmeldetag: 11.05.2007
(51) Int. Cl.: A61K 6/00, A61K 6/083

(54) **POLYMERISIERBARES DENTALMATERIAL**
POLYMERIZABLE DENTAL MATERIAL
MATÉRIAU DENTAIRE POLYMÉRISABLE

(30) Priorität: 30.06.2006 EP 06013589
(43) Veröffentlichungstag der Anmeldung: 18.03.2009
(73) Patentinhaber: Ernst Mühlbauer GmbH & Co.KG, 25870 Norderfriedrichskoog (DE)
(72) Erfinder: LÜCK, Rainer, 25436 Tornesch (DE)
(74) Vertreter: Glawe, Delfs, Moll
(86) Internationale Anmeldenummer: PCT/EP2007/004207
(87) Internationale Veröffentlichungsnummer: WO 2008/000313

(56) Entgegenhaltungen:
- EP-A2- 0 923 924
- WO-A-2007/140440
- WO-A2-2007/140440
- US-A- 5 707 611

## Beschreibung

Die Erfindung betrifft ein polymerisierbares Dentalmaterial und die Verwendung von Salzen einer CH-aciden Verbindung.

Chemisch härtende polymerisierbare Dentalmaterialien (in der Literatur häufig auch selbsthärtende oder autokatalysierte Dentalmaterialien genannt) enthalten polymerisierbare Monomere, deren Polymerisation durch primär gebildete Radikale ausgelöst wird. Die Bildung dieser Radikale erfolgt durch die Reaktion eines geeigneten Initiatormoleküls, das bei Raumtemperatur allein eine ausreichende Lagerstabilität besitzt, mit einem Coinitiator. Da diese Reaktion sofort nach Zusammentreffen von Initiator und Coinitiator beginnt, müssen zur Lagerung der Dentalmaterialien beide Komponenten des Initiatorsystems getrennt untergebracht werden. Es resultieren daher, im Unterschied zu lichthärtenden Materialien, bei denen die Radikale erst durch Bestrahlung mit der blauen Komponente des sichtbaren Lichtes gebildet werden, Mehr-Komponenten-Systeme. Die Komponenten werden erst unmittelbar vor der Verarbeitung des Materials miteinander in Kontakt gebracht und innig miteinander vermischt. Diese Vermischung kann dabei entweder von Hand mit einem Mischspatel oder durch selbstanmischenden Systeme (Doppelkartusche mit statischen oder dynamischen Mischkanülen) erfolgen.

Ein im Stand der Technik bei chemisch härtenden Dentalmaterialien am häufigsten verwendetes Initiatorsystem besteht aus einem zumeist aromatischen Amin und einem organischen Peroxid, wie z.B. in DE-C-97 50 72 beschrieben. Die benötigten Radikale werden bei diesem System über eine Redoxreaktion zwischen Amin und Peroxid gebildet.

Ein wesentlicher Nachteil der Amin/Peroxidsysteme besteht in der allgemein schlechten Farbstabilität. Ursache dafür sind bei Parallel-, Neben- und Folgereaktionen gebildete Produkte der Initiatorkomponenten, die aufgrund ihrer Struktur häufig gefärbt sind. Solche gefärbten Verbindung können bei der Radikalbildung entstehen, die während der Lagerung der Pasten gebildet werden oder im ausgehärteten Material zum Beispiel durch Einwirkung von sichtbarem oder UV-Licht beobachtet werden (z.B. A. Schmidt: "Kaltpolymerisate: Ein Bericht über ihre Eigenschaften, Einsatzmöglichkeiten und Vorteile", Dentallabor 11 [1970] S. 17-22). Dieser Nachteil kann bei den Amin/Peroxid-Systemen auch durch den durchaus üblichen Zusatz spezieller Licht- und UV-Stabilisatoren nicht behoben werden. Für hochästhetische Versorgungen sind diese Verfärbungen für den Patienten störend bzw. nicht akzeptabel. Daher wird für Versorgungen im Frontzahnbereich häufig auf sehr viel aufwendigere und teurere Keramik (Veneers, Kronen, Brücken, u.ä.) zurückgegriffen.

Ein weiterer Nachteil der Amin/Peroxid-Systeme besteht in der toxischen und allergieauslösenden Wirkung der Komponenten des Startersystems und deren Reaktions- und Abbauprodukten. Während des Härtungsprozesses kann eine direkte toxische Wirkung von diesen Komponenten ausgehen. Auch nach der Härtung können entsprechende nicht einpolymerisierte Moleküle durch den sauren Speichel ausgewaschen werden. Bei einer Reihe von Patienten resultieren allergische Reaktionen, die die Anwendung von Kunststoffen einschränken oder ausschließen. In Einzelfällen kann die toxische Wirkung einen anaphylaktischen (allergischen) Schock auslösen, der durchaus lebensbedrohliche Formen annehmen kann.

Weiterhin problematisch ist der Temperaturanstieg bei der Polymerisation aufgrund der exothermen Reaktionsprozesse. Amin/Peroxid gestartete Systeme polymerisieren vergleichsweise schnell und besitzen so am Gelpunkt bereits einen sehr hohen Vernetzungsgrad (Umsatz an Doppelbindungen), was eine relativ hohe Wärmemenge aus der exothermen Reaktion freisetzt. Hohe Temperaturmaxima sind die Folge. Eine zu hohe Temperatur kann aber zu Pulpaschädigungen bis hin zum Absterben des Zahnes führen.

Ein alternatives Initiatorsystem, das eine günstigere Temperaturentwicklung und eine deutlich bessere Farbstabilität besitzt, benutzt CH-acide Verbindungen in Kombination mit zweiwertigen Übergangsmetallionen und Chloridionen. Entsprechende CH-acide Verbindungen wurden intensiv von H. Bredereck und seinen Mitarbeitern untersucht (H. Bredereck et all: "Über CH-Aktive Polymerisationsinitiatoren - XIII. Mitt. Polymerisationen und Polymerisationsinitiatoren", die Makromolekulare Chemie 92 [1966] S. 70-90; H. Bredereck et all: "Polymerisationen und Polymerisationsinitiatoren - 16. Einfluß von Thio-Gruppen in Barbitursäurederivaten auf die Polymerisationsauslösung von Methacrylsäure-methylester", die Makromolekulare Chemie 176 [1975'] S. 1713-1723). Von den CH-aciden Verbindungen haben sich im Dentalbereich die Barbitursäurederivate als günstig erwiesen. Sie sind in hohen Ausbeuten und Reinheiten darstellbar, industriell verfügbar (Chemische Fabrik Berg GmbH, Mainthalstr. 3, D-06749 Bitterfeld) und erlauben durch ihre Reaktionskinetik die Realisierung interessanter Eigenschaften.

Die Synthese der Barbitursäurederivate ist z.B. aus E. Fischer und A. Dilthey: "Über c-Dialkylbarbitursäuren und über die Ureide der Dialkylessigsäuren", Ann. 335 [1904] S. 335) bekannt und beschreibt die alkalische Kondensation von Derivaten des Malonsäurediethylesters mit N-substituiertem Harnstoff in Natriumalkoholat. Die dabei erhaltenen Natriumsalze der Barbitursäurederivate werden anschließend durch die Zugabe einer Säure, z.B. von Salzsäure, in die Barbitursäurederivate überführt.

Bei dem auf Barbitursäure bzw. deren Derivaten basierenden Initiatorsystem müssen die Barbitursäurederivate von den polymerisierbaren Monomeren getrennt aufbewahrt werden. Dies liegt darin begründet, dass CH-acide Verbindungen wie die Derivate der Barbitursäure bereits ohne die Mitwirkung von Cu(II)- und Clorid-Ionen durch Autoxidation durch Luftsauerstoff Hydroperoxide bilden. Diese Hydroperoxide zerfallen unter Bildung von Radikalen, welche die Polymerisation der reaktiven Monomere initiieren, so dass es binnen kurzer Zeit zur spontanen Polymerisation kommt. Dieser spontane Polymerisationsprozess kann durch Zugabe von Stabilisatoren für kurze Zeit (im Bereich von wenigen Stunden) verzögert oder unterdrückt werden, nicht hingegen über einen längeren Zeitraum, wie es bei lagerstabilen Systemen erwünscht ist.

Stand der Technik ist hier der Ersatz reaktionsfähiger Harze in der Initiatorpaste durch solche, die unter dentalen Bedingungen durch CH-acide Verbindungen nicht zur Polymerisation gebracht werden können, oder durch Verbindungen, die keine Doppelbindungen enthalten (z.B. Polyethylenglycol).

Die erforderliche räumliche Trennung von polymerisierbaren Monomeren und CH-aciden Barbitursäurederivaten begrenzt den Anteil der polymerisierbaren Monomere im Dentalmaterial. Für fließfähige Materialien, die vorrangig aus herkömmlichen Doppelkartuschensystemen appliziert werden, kann die Zugabe nicht reaktiver Monomerer durch die Verringerung der Zugabe dieser Pastenkomponente (Mischungsverhältnisse von 2:1, 4:1 und 10:1) zumindest vermindert werden. Aus diesen Gründen werden Barbitursäurederivate in Verbindung mit Cu²⁺ und Cl⁻ derzeit ausschließlich bei den fließfähigen automatisch dosierenden und anmischenden provisorischen Kronen- und Brückenmaterialien eingesetzt. Allerdings wirkt auch die geringere Menge durch die Initiatorpaste zugesetzter nicht polymerisierender Monomere als Trennmittel, was zu einer Verschlechterung der mechanischen Eigenschaften (Druckfestigkeit, Biegefestigkeit, Härte usw.) und zu einer Zunahme der Schmierschicht führt.

Hochviskose Materialien, wie z.B. modellierbare chemisch härtender Füllungskomposite sind aus Kartuschensystemen nicht ausbringbar und nicht automatisch anmischbar. Sie werden in Drehspritzen oder Dosen untergebracht und von Hand angemischt. Die Dosierung erfolgt durch den Zahnarzt ausschließlich nach Augenmaß. Dosierhilfen haben sich bisher nicht durchgesetzt. Da gleiche Materialialmengen besser einschätzbar sind, als z.B. 10:1 werden diese Materialien ausschließlich im Mischungsverhältnis von 1:1 angewendet. Dieses Mischungsverhältnis ist vom Zahnarzt besser einschätztbar, als ungleiche Verhältnisse und führt so zu wesentlich kleineren Dosierfehlern und damit zu besseren Materialqualitäten. Obwohl die CH-aciden Barbitursäurederivate zu wesentlich besseren Farbstabilitäten führen, als Amin/Peroxid-Systeme, bleiben diese Systeme Füllungs- und Verblendmaterialien wegen der hohen Zugabe nicht polymerisierender Monomere und der damit einhergehenden Verschlechterung der mechanischen Eigenschaften bisher verschlossen.

EP0923924 beschreibt ein zahnmedizinisches Klebstoffset, das die folgenden Komponenten umfasst:
(A) ein radikalpolymerisierbares Monomer mit einer Säuregruppe im Molekül,
(B) fakultativ ein radikalpolymerisiserbares Monomer ohne Säuregruppe, das nicht oder nur schwer in Wasser löslich ist,
(C) ein Fotosensibilisator (C1) und /oder ein Peroxid (C2),
(D) ein wasserlösliches organisches Lösungsmittel,
(E) eine organische Sulfinsäure und oder ein Salz davon oder eine Barbitursäure und/oder ein Derivat davon, und
(F) Wasser,
wobei dir Komponente (E) an einen Applikator angeheftet ist, von den übrigen in einem Gefäss befindlichen Komponenten räumlich getrennt ist und mit diesen erst unmittelbar vor der Anwendung kontaktiert und vermischt wird.

Der vorliegenden Erfindung liegt die Aufgabe zu Grunde, ein Initiatorsystem für polymerisierbare Dentalmaterialien zu schaffen, dass die aus dem Stand der Technik bekannten Nachteile vermeidet, und darüber hinaus auch in 1:1 dosierenden Materialien wie Füllungs- und Verblendkunststoffen einsetzbar ist.

Die Erfindung löst diese Aufgabe durch ein polymerisierbares Dentalmaterial aus wenigstens zwei Komponenten, dass die folgenden Komponenten umfasst:
Komponente 1 enthaltend
   a) das Salz einer CH-aciden Verbindung, wobei die CH-acide Verbindung eine radikalische Polymerisation auslösen kann,
Komponente 2 enthaltend
   b) eine Säure, deren Säurestärke größer ist als die der in der in Komponente 1 als Salz vorliegenden CH-aciden Verbindung,
wobei die Komponente 1 und die Komponente 2 des polymerisierbaren Dentalmaterials radikalisch polymerisierbare Monomere enthalten.

Kern der Erfindung ist, das im Unterschied zu den Startersystemen basierend auf CH-aciden Verbindungen aus dem Stand der Technik eine Vorstufe des aktiven Startermoleküls, nämlich ein Salz der CH-aciden Verbindung, eingesetzt wird. Die CH-acide Verbindung wird erst nach der Zugabe einer Säure, deren Säurestärke größer ist als die der als Salz vorliegenden CH-aciden Verbindung gemäß der Regel "Salz einer schwachen Säure + starke Säure ergibt Salz einer starken Säure + schwache Säure", freigesetzt und kann anschließend als Startermolekül für den Polymerisationsprozess der Monomere fungieren.

Die Erfindung hat erkannt, dass im Unterschied zu den CH-aciden Verbindungen, wie sie in Startersystemen des Standes der Technik verwendet werden, das Salz der CH-aciden Verbindung auch über längere Zeiträume lagerstabil ist. Damit wird die Initiatoraktivität für die Polymerreaktion der polymerisierbaren Monomere auch bei längerer Lagerung der Komponenten des polymerisierbaren Dentalmaterials sichergestellt. Gemäß einer bevorzugten Ausführungsform der Erfindung sind die Pasten, aus denen das polymerisierbare Dentalmaterial angemischt wird, und insbesondere die das Salz der CH-aciden Verbindung enthaltende Paste, mehr als 3 Monate, vorzugsweise mehr als 6 Monate, besonders bevorzugt mehr als 24 Monate farb- und/oder lagerstabil ist.

Die Erfindung hat des Weiteren erkannt, dass man ausgehend von dem erfindungsgemäßen Startersystem, gemäß einer bevorzugten Ausführungsform der Erfindung in beide Komponenten des Dentalmaterials polymerisierbare Monomere einbringen kann. Damit kann die eingangs erläuterte unerwünschte Beschränkung der Menge an polymerisierbaren Monomeren im polymerisierbaren Dentalmaterial, d. h. der Anteil der Polymermatrix im Dentalmaterial, aufgehoben werden. Daraus ergeben sich auch vorteilhafte mechanische Eigenschaften des polymerisierten Dentalmaterials, da die Menge der nicht reaktiven Monomere (z.B. Monomere mit nicht reaktiven Doppelbindungen oder Verbindungen, die keine Doppelbindungen enthalten), die üblicherweise aus Gründen der Handhabbarkeit der Initiatorpaste zugefügt werden (z. B. zur Einstellung pastöser Eigenschaften um die Paste in Kartuschensystemen verwenden zu können), verringert bzw. ganz darauf verzichtet werden kann. Es ist bekannt, das nicht einpolymerisierende Harze oder Füllstoffe in den Basis- und Initiatorpasten wie Trennmittel wirken und je nach Gehalt die mechanischen Eigenschaften nachteilig beeinflussen. Dieser Effekt wird bei der Verwendung des erfindungsgemäßen Startersystems weitestgehend vermieden, bei dem gemäß einer bevorzugten Ausführungsform der Erfindung die Monomere beider Komponenten bei deren Mischung radikalisch polymerisieren.

Erfindungsgemäß bevorzugte radikalisch polymerisierbare Monomere sind aus der Gruppe der Acrylsäureester und Methacrylsäureester ausgewählt.

Das erfindungsgemäße polymerisierbare Dentalmaterial enthält vorzugsweise insgesamt über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere. Gemäß einer bevorzugten Ausführungsform der Erfindung enthält die Komponente 1 des polymerisierbaren Dentalmaterials über 50 Gew-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere. Gemäß einer bevorzugten Ausführungsform der Erfindung enthält die Komponente 1 und/oder die Komponente 2 oder das polymerisierbare Dentalmaterial insgesamt keine nicht radikalisch polymerisierbaren Monomere.

Das erfindungsgemäße polymerisierbare Dentalmaterial kann die Komponente 1 in einer ersten Paste und die Komponente 2 in einer zweiten Paste enthalten und in einem vorgesehenen Mischungsverhältnis beider Pasten von 1:10 oder größer, vorzugsweise 1:4 oder größer, weiter vorzugsweise 1:2 oder größer, besonders bevorzugt 1:1 angemischt werden.

Als Salz der CH-aciden Verbindung der Komponente 1 sind insbesondere Salze der α-Benzoyl-proprionitrile, α-Cyan-carbonsäureester und -amide, cyclischer β-Oxonitrile, β-Diketone, cyclischer β-Diketone, cyclischer β-Oxocarbonsäureester, cyclischer β-Oxo-lactone, Malonsäure, Malonsäurederivate, Pyrazolderivate, Barbitursäure oder Barbitursäurederivate geeignet.

Das Salz der CH-aciden Verbindung ist vorzugsweise ein Salz ausgewählt aus der Gruppe bestehend aus einwertigen und zweiwertigen Salzen der Alkali- und Erdalkaliionen. Das Salz der CH-aciden Verbindung kann beispielsweise ein Natriumsalz sein.

Als Säure der Komponente 2 im Sinne vorliegender Erfindung kann eine organische oder anorganische Säure verwendet werden, sofern deren Säurestärke größer ist als die der in der Komponente 1 als Salz vorliegenden CH-aciden Verbindung.

Als anorganische Säuren kommen nicht oxydierende Säuren wie z.B. Salzsäure oder Phosphorsäure in Betracht.

Besonders geeignete organische Säuren sind Monocarbonsäuren, ausgewählt aus der Gruppe bestehend aus Ameisensäure, Essigsäure und Benzoesäure bzw. Derivate dieser Säuren oder Dicarbonsäuren, ausgewählt aus der Gruppe bestehend aus Oxalsäure, Malonsäure, Succinsäure, Adipinsäure, Pimelinsäure, Azelainsäure, Sebacinsäure, Maleinsäure, Fumarsäure, Sorbinsäure, Phthalsäure und Terephthalsäure bzw. Derivate dieser Säuren oder Tricarbonsäuren, ausgewählt aus der Gruppe bestehend aus Hemimellithsäure, Trimellithsäure, Trimesinsäure, Agaricinsäure, Citronensäure, 1,2,3-Propantricarbonsäure bzw. Derivate dieser Säuren oder Multicarbonsäuren, ausgewählt aus der Gruppe bestehend aus Pyromellithsäure und Mellithsäure bzw. Derivate dieser Säuren oder Polycarbonsäuren, ausgewählt aus der Gruppe bestehend aus Polyacrylsäure und Polymethacrylsäure bzw. Derivate dieser Säuren.

Der sich auf das Dissoziationsgleichgewicht in einem wässrigen Medium beziehende pKₐ-Wert kann dabei als ein Maß für die CH-Acidität zumindest mit herangezogen werden. Eine Auswahl an organischen Säuren im Sinne vorliegender Erfindung, deren pKₐ-Wert kleiner als der pKₐ-Wert der Barbitursäure (4,01) ist, sind beispielsweise 2,5-Dihydroxybenzoesäure (2,97), Fumarsäure (3,03), Maleinsäure (1,83), Phtalsäure (2,89), Salicylsäure (2,97), 2,4,6-Trihydroxybenzoesäure (1,68) und Zimtsäure (3,89).

Das erfindungsgemäße polymerisierbare Dentalmaterial kann in mindestens einer der Komponenten des polymerisierbaren Dentalmaterials Übergangsmetallkationen, vorzugsweise Cu²⁺-Ionen, und zur Radikalbildung geeignete Anionen, vorzugsweise Halogenid-Ionen, weiter vorzugsweise Chlorid-Ionen, enthalten, die den Polymerisationsprozess initiieren, steuern und beschleunigen. Die fakultativ zum Startersystem gehörenden Übergangsmetallkationen, vorzugsweise Cu²⁺-Ionen, und zur Radikalbildung geeigneten Anionen, vorzugsweise Halogenid-Ionen, weiter vorzugsweise Chlorid-Ionen, liegen vorzugsweise in der Basispaste (Komponente 1) des anspruchsgemäßen polymerisierbaren Dentalmaterials vor, können aber je nach Erfordernis auch der Inititiatorpaste (Komponente 2) zugesetzt werden.

Das erfindungsgemäße Dentalmaterial kann in mindestens einer der Komponenten Füllstoffe enthalten. Bei den erfindungsgemäß eingesetzten Füllstoffen handelt es sich bevorzugt um nano- und/oder mikroskalige (teilweise röntgenopake) Füllstoffe, vorzugsweise um Glaspulver, Glaskeramikpulver, Metall-, Halbmetall- oder Mischmetalloxide, Silikat-, Nitrid-, Sulfat-, Titanat-, Zirkonat-, Stannat-, Woframat-, Siliciumdioxid-Verbindungen oder eine Mischung aus diesen Verbindungen oder sphärische Füllstoffe, Quarzpulver oder eine Mischung aus diesen Pulvern oder gefüllte oder ungefüllten Splitterpolymerisate und/oder Perlpolymerisate. Bei den erfindungsgemäß eingesetzten nanoskaligen Füllstoffen handelt es sich besonders bevorzugt um Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Der Füllstoff kann gemäß einer bevorzugten Ausführungsform der Erfindung ein oberflächenmodifizierter Füllstoff, vorzugsweise eine organisch oberflächenmodifizierter Füllstoff sein. Der Füllstoff kann nach seiner Oberflächenmodifikation, beispielsweise einer Silanisierung, auf seiner Oberfläche funktionelle Gruppen, beispielsweise reaktive Methacrylatgruppen besitzen, die mit den Monomeren chemisch, vorzugsweise radikalisch, reagieren können oder eine hohe Affinität zu der aus den Monomeren gebildeten Polymermatrix aufweisen.

Das erfindungsgemäße Dentalmaterial kann zur Einstellung bestimmter Eigenschaften zusätzlich so genannte Additive bzw. Modifikatoren enthalten. Die Allgemeinheit nicht einschränkend sind nachfolgend einige Beispiele genannt: anorganische und/oder organische Farbpigmente bzw. Farbstoffe, Stabilisatoren (wie z. B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z. B. Polethylenglykole, Polypropylenglykole, ungesättigte Polyester, Phthalate, Adipate, Sebacate, Phosphorsäureester, Phosphonsäureester und/oder Zitronensäureester), ionenabgebende Substanzen, insbesondere solche die Fluoridionen freisetzen (wie z. B. Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester).

Das erfindungsgemäße Dentalmaterial kann für die prothetische, konservierende und präventive Zahnheilkunde verwendet wenden. Ohne Anspruch auf Vollständigkeit seien stellvertretend einige Anwendungsbeispiele genannt: Füllungsmaterial, Stumpfaufbaumaterial, Befestigungsmaterial, provisorisches und permanentes Kronen- und Brückenmaterial, Bonding-Materialien, zahntechnischer Werkstoff zur Herstellung von Inlays, Onlays, Verblendschalen, künstlichen Zähnen, Modellmaterialien und Fissuren- und Wurzelkanal-Versiegelungsmaterial.

Die Erfindung wird nachfolgend ohne Einschränkung der Allgemeinheit anhand von Ausführungsbeispielen erläutert.

### Beispiel 1: Synthese des Natriumsalzes der 1,3,5-Trimethylbarbitursäure

Die Synthese wurde von A.C.Cope et all.: "1,3-Dimethyl-5-alkyl Barbituric Acids": J.Amer.Chen.Soc. 63 365 (1941)beschrieben. 0,1 mol (=17,420g) Methylmalonsäurediethyl-ester wurden zu 97,214 g 21%-ige Natriumalkoholatlösung in Ethanol (= 0,3 mol Natriumalkoholat) gegeben und beide Komponenten innig miteinander vermischt. Dabei entstehen Natrium-methylmalonsäure-diethylester und Ethanol. Anschließend wurde 0,1 mol (= 8,811 g) N, N-Dimethylharnstoff in 15 ml Ethanol (p.a.) gelöst und zur Lösung langsam zugetropft, wobei das Natriumsalz der 1,3,5-Trimethylbarbitursäure entsteht. Anschließend wurde der Ansatz 11,5 Stunden unter Rückfluß gekocht. 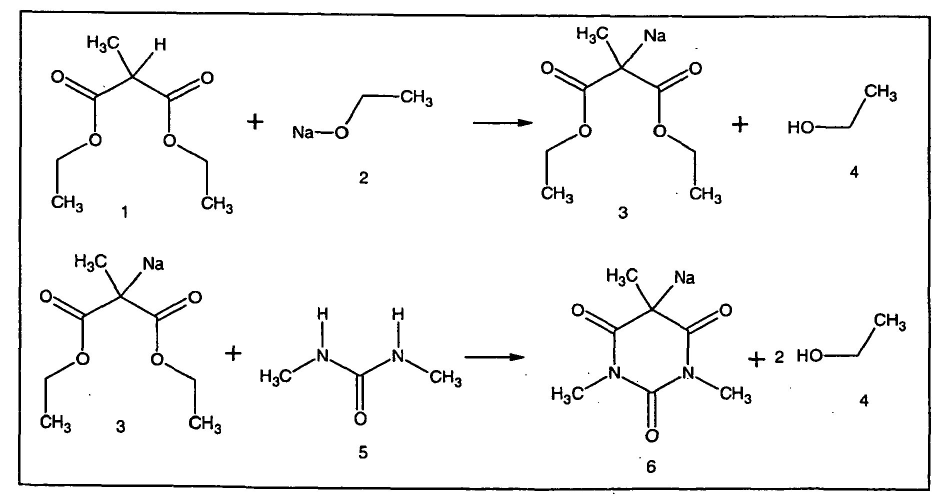
1 = Methylmalonsäure-diethylester
2 = Natriumalkoholat
3 = Natrium-methylmalonsäure-diethylester
4 = Ethanol
5 = N,N-Dimethylharnstoff
6 = 1,3,5-Trimethylbarbitürsäure-Natriumsalz

Die Lösung wurde am Rotationsverdampfer bis zur Trockne eingeengt und der Rückstand mit 100 ml deionisiertem Wasser aufgenommen. Danach wurde 5 Mal mit je 20 ml Ether ausgeschüttelt. Die wässrige Phase wurde am Rotationsverdampfer erneut bis zur Trockne eingeengt und anschließend in eine Filter-nutsche überführt. Hier wurde der Rückstand mit Isopropanol gewaschen, bis das abgesaugte Isopropanol keine Färbung mehr aufwies. Da der Feststoff immer noch leicht bräunlich gefärbt war, wurde mit wenig Ethanol (p.a.) gewaschen, bis auch das Ethanol keine Färbung mehr aufwies.
Die Reinheit wurde mittels HPLC mit 99,59% bestimmt. Die Ausbeute betrug 65,30%.

### Beispiel 2: Herstellung und Lagerstabilität der Initiatorpaste

Es wurden drei unterschiedliche Initiatorpasten hergestellt und auf ihre Lagerstabilität bei Raumtemperatur und bei 40°C untersucht:
- Paste 1:: Paste mit Polyethylenglycol mit mittlerem Molekulargewicht von 400 g/mol (PEG 400) als unreaktive Harzkomponente und mit Dentalglas gefüllt, das mit methacrylatgruppenfreiem Silan oberflächenbehandelt war und 1,3,5- Trimethylbarbitursäure als Startermolekül enthielt.
- Paste 2:: Paste mit Methacrylaten und mit Dentalglas gefüllt, das mit Methacrylatgruppen tragendem Silan oberflächenbehandelt war und 1,3,5-Tri- methylbarbitursäure als Startermolekül enthielt.
- Paste 3:: Paste mit Methacrylaten und mit Dentalglas gefüllt, das mit Methacrylatgruppen tragendem Silan oberflächenbehandelt war und das Natrium- salz der 1,3,5-Trimethylbarbitursäure als Startermolekül enthielt.

Die folgende Tabelle zeigt die Rezepturen der Pasten. Dabei war die zugegebene Startermolekülmenge so berechnet, dass alle drei Pasten gleiche Molzahlen enthielten.

| Bestandteil | Paste 1 | Paste 2 | Paste 3 |
|---|---|---|---|
| | Gew.-% | Gew.-% | Gew.-% |
| | | | |
| Bis-GMA | - | 37,2179 | 36,9790 |
| TEDMA | - | 16,7211 | 16,6138 |
| PEG 400 | 53,9390 | - | - |
| Aerosil R812 | 1,5000 | 1,5000 | 1,4904 |
| Dentalglas sil. ohne Methacrylatgruppen | 39,9350 | - | - |
| Dentalglas sil. mit Methacrylatgruppen | - | 39,9350 | 39,6785 |
| 1,3,5-Trimethylbarbitursäure | 4,6260 | 4,6260 | - |
| 1,3,5-Trimethylbarbitursäure Natriumsalz | - | - | 5,2383 |

Um eine Aussage über die Lagerstabilität der Pasten zu erhalten, wurden die Pasten bei Raumtemperatur und bei 40°C eingelagert und in regelmäßigen Abständen auf polymerisierte Anteile untersucht. Während die Paste 2 bereits nach 90 min vollständig auspolymerisiert war, zeigen die Pasten 1 und 3 auch nach mehr als 6 Monaten noch keinerlei Anzeichen von Polymerisation. Das zeigt, dass Pasten mit reaktiven Monomeren und 1,3,5-Trimethylbarbitursäure als aktives Startermolekül nicht lagerstabil sind, während bei Einsatz des Natriumsalzes der 1,3,5-Trimethylbarbitursäure als Vorstufe des aktiven Startermoleküls 1,3,5-Trimethylbarbitursäure lagerstabile Pasten resultieren.

### Beispiel 3: Untersuchung der Reaktionsfähigkeit der Initiatorpasten aus Beispiel 2

Um die Reaktionsfähigkeit der im Beispiel 2 hergestellten Initiatorpasten zu untersuchen, wurden diese von Hand mit der Basispaste des Produktes Luxatemp Automix A2 der Fa. DMG Hamburg im Verhältnis 10 : 1 (10 Teile Basispaste, 1 Teil Initiatorpaste) angemischt und die Erhärtungszeit bestimmt.

Mit der Polyethylenglykolhaltigen Paste 1 begann die Erhärtung nach 1:40 min. und war nach 3:20 min. abgeschlossen. Das bedeutet, dass diese Paste lagerstabil und reaktionsfähig ist.

Bei Verwendung der Initiatorpaste 2 ergibt sich ein Erhärtungsbeginn von 1:50 min. die Erhärtung ist hier ebenfalls nach ca. 3:20 min. abgeschlossen. D.h. die Paste besitzt eine ausreichende Reaktivität, ist aber, wie Beispiel 2 zeigte, nicht lagerstabil. Sie ist daher als Initiatorpaste nicht einsetzbar.

Die Initiatorpaste 3, die reaktive Methacrylgruppen und das Natriumsalz der 1,3,5-Trimethylbarbitursäure enthält, erhärtete nach dem Anmischen mit der oben genannten Basis erwartungsgemäß nicht. Deshalb wurde dem angemischten System (0,6g Basispaste + 0,06g Initiatorpaste) 1 Tropfen 32%-ige Salzsäure zugesetzt. Nach 55 min. polymerisierte das Material.

### Beispiel 4: Reaktion der Initiatorpaste 3 aus Beispiel 2 mit Salzsäure bzw. einer Salzsäure enthaltenden Basispaste

Die Initiatorpaste 3 aus Beispiel 2, die das Natriumsalz der 1,3,5-Trimethylbarbitursäure und reaktive Methacrylatmonomere enthält, wurde mit einem Tropfen Salzsäure gemischt. Nach ca. 90 Minuten war die Paste ausgehärtet.

In einem zweiten Versuch wurde der Initiatorpaste 3 aus Beispiel 2 neben dem Tropfen Salzsäure die reaktive Methacrylatmonomere und Coinitiatoren enthaltende Basispaste des Produktes Luxatemp Automix der Fa. DMG Hamburg im Verhältnis 1:1 zur Initiatorpaste zugemischt. Die erhaltene Paste polymerisierte nach 20 Minuten.

Die Versuche belegen, dass das Natriumsalz des Barbitursäurederivats *in situ* in die freie Säure nach Zugabe der Salzsäure überführt wird und nach Aufbau der CH-Acidität die Pasten infolge der sich anschließenden Autoxydation nach kurzer Zeit polymerisieren. Die Versuche zeigen des Weiteren, dass durch in der Basispaste enthaltene Coinitiatoren, beispielsweise Alkylammoniumchloride und Cu(II)-Verbindungen, wie sie in der Basispaste des Produktes Luxatemp Automix enthalten sind, die Polymerisationszeiten erheblich verkürzen können.

### Beispiel 5: Reaktion der Initiatorpaste 3 aus Beispiel 2 mit organischen Säuren

Es wurden eine Auswahl an organischen Säuren, deren pKₐ-Wert unter dem der Barbitursäure liegt, auf Ihre Fähigkeit geprüft, das Natriumsalz der Paste 3 gemäß Beispiel 2 in die freie 1,3,5-Trimethylbarbitursäure zu überführen. Als Maß für die Überführbarkeit diente die Erhärtungszeit des Pastengemisches. Die Konzentration der verwendeten organischen Säuren wurde so berechnet, dass sie äquimolar zu der in der Initiatorpaste eingesetzten Molzahl des Startermoleküls bei einer Anmischung von 10:1 war. Bei der Anmischung der Initiatorpaste mit den nachfolgend genannten organischen Säuren
2,5-Dihydroxybenzoesäure,
Fumarsäure,
Maleinsäure,
Phtalsäure,
Salicylsäure,
2,4,6-Trihydroxybenzoesäure und
Zimtsäure
war innerhalb von 30 Minuten eine Vergelung, d.h. Polymerisation, zu beobachten.

Beispielhaft sei dies nachfolgend für die Reaktivität der Initiatorpaste 3 aus Beispiel 2 nach Zugabe von Fumarsäure bzw. 2,5-Dihydroxybenzoesäure beschrieben.

0,5g der oben genannten Basispaste wurden mit 0,05g der Initiatorpaste 3 aus Beispiel 2 vermischt und 1 Tropfen in Hydroxyethylmethacrylat gelöste Fumarsäure (0,1g in 10 ml) zugegeben. Das Material polymerisierte nach 6 Minuten.

In einem weiteren Versuch wurde nun die Fumarsäurelösung in die Basispaste eingearbeitet. Dabei wurde ein Molverhältnis von Fumarsäure: 1,3,5-Trihydroxybarbitursäurenatriumsalz von 1:1 (nach Anmischung der Pasten im Verhältnis von 10:1) eingestellt. Das Material polymerisierte nach 19 Minuten.

In einem weiteren Versuch wurde die in Hydroxyethylmethacrylat besser lösliche 2,5-Dihydroxybenzoesäure eingesetzt. Dazu wurde 0,1g der 2,5-Dihydroxybenzoesäure in 1 ml Hydroxyethylmethacrylat gelöst. 1 Tropfen der Lösung (0,0248g) wurden in 0,5g der oben genannten Basispaste eingearbeitet. Anschließend wurden 0,05g der Initiatorpaste 3 aus Beispiel 2 zugegeben und gründlich durchmischt. Nach 9 min. war das Pastengemisch erhärtet.

### Beispiel 6 (erfindungsgemäß)

Um die Reaktionsfähigkeit und die Biegefestigkeit als Maß für die mechanische Festigkeit des erfindungsgemäßen Dentalmaterials zu untersuchen, wurde die nachfolgend spezifizierte Initiatorpaste 4 hergestellt und mit der Basispaste aus Beispiel 3, in die 1,53 Gew.-% 2,5-Dihydroxybenzoesäure eingearbeitet wurde, im Verhältnis 1:1 angemischt.

| Bestandteil | Paste 4 |
|---|---|
| | Gew.-% |
| | |
| Bis-GMA | 38,5 |
| TEDMA | 17,1 |
| PEG 400 | - |
| Aerosil R812(oberflächenbehandelte Pyrogene Kieselsäure) | 1,6 |
| Dentalglas sil. mit Methacrylatgruppen (D50: 1,5 µm) | 42,1 |
| 1,3,5-Trimethylbarbitursäure | - |
| Na-1,3,5-Trimethylbarbiturat | 0,7 |

Die Erhärtung begann nach 2:30 min und war nach 5:30 min. abgeschlossen. Die mittlere Biegefestigkeit betrug 72,11 MPa (+/- 6,00, 10 Messungen). Die maximale Biegefestigkeit betrug 81,97 MPa.

### Beispiel 7 (Vergleichsbeispiel)

Um die Biegefestigkeit eines nicht erfindungsgemäßen Dentalmaterials zu untersuchen, wurde die nachfolgend spezifizierte Initiatorpaste 5 hergestellt und mit der Basispaste aus Beispiel 3 im Verhältnis 1:1 angemischt.

| Bestandteil | Paste 5 |
|---|---|
| | Gew.-% |
| | |
| Bis-GMA | - |
| TEDMA | - |
| PEG 400 | 56,1 |
| Aerosil R812(oberflächenbehandelte Pyrogene Kieselsäure) | 1,6 |
| Dentalglas sil. ohne Methacrylatgruppen (D50: 1,5 µm) | 41,5 |
| 1,3,5-Trimethylbarbitursäure | 0,8 |
| Na-1,3,5-Trimethylbarbiturat | - |

Die mittlere Biegefestigkeit des ausgehärteten Dentalmaterials betrug 16,89 MPa (+/- 2,25, 10 Messungen) und die maximale Biegefestigkeit betrug 19,03 MPa.

Die Biegefestigkeit des Dentalmaterials des erfindungsgemäßen Beispiels 6 mit einer mittleren Biegefestigkeit von 72,11 MPa und einer maximale Biegefestigkeit 81,97 MPa lag damit deutlich über den entsprechenden Werten des Vergleichsbeispiels 7 und belegt die extreme Belastbarkeit des erfindungsgemäßen Dentalmaterials.

## Patentansprüche

1. Polymerisierbares Dentalmaterial aus wenigstens zwei Komponenten, **dadurch gekennzeichnet, dass** es die folgenden Komponenten umfasst:
Komponente 1 enthaltend
a) das Salz einer CH-aciden Verbindung, wobei die CH-acide Verbindung eine radikalische Polymerisation auslösen kann,
Komponente 2 enthaltend
b) eine Säure, deren Säurestärke größer ist als die der in der in Komponente 1 als Salz vorliegenden CH-aciden Verbindung,
wobei die Komponente 1 und die Komponente 2 radikalisch polymerisierbare Monomere enthalten.

2. Polymerisierbares Dentalmaterial nach Anspruch 1, **dadurch gekennzeichnet, dass** das polymerisierbare Dentalmaterial insgesamt über 50 Gew.-%, vorzugsweise über 60 Gew.-%, weiter vorzugsweise über 70 Gew.-%, weiter vorzugsweise über 80 Gew-%, weiter vorzugsweise über 90 Gew.-%, weiter vorzugsweise über 95 Gew.-%, weiter vorzugsweise über 98 Gew-% radikalisch polymerisierbare Monomere enthält.

3. Polymerisierbares Dentalmaterial nach Anspruch 1 oder 2, **dadurch gekennzeichnet, dass** die Komponente 1 über 50 Gew-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.% radikalisch polymerisierbare Monomere enthält.

4. Polymerisierbares Dentalmaterial nach einem der vorangehenden Ansprüche, **dadurch gekennzeichnet, dass** die Komponente 1 und/oder die Komponente 2 oder das polymerisierbare Dentalmaterial insgesamt keine nicht radikalisch polymerisierbaren Monomere enthält.

5. Polymerisierbares Dentalmaterial nach einen der vorangehenden Ansprüche, **dadurch gekennzeichnet, dass** die Monomere in Komponente 1 mit den Monomeren in Komponente 2 bei der Mischung beider Komponenten radikalisch polymerisieren können und weiter vorzugsweise, dass die radikalisch polymerisierbaren Monomere ausgewählt sind aus der Gruppe bestehend aus Acrylsäureester und Methacrylsäureester.

6. Polymerisierbares Dentalmaterial nach einem der vorangehenden Ansprüche; **dadurch gekennzeichnet, dass** die Komponente 1 in einer ersten Paste und die Komponente 2 in einer zweiten Paste enthalten ist und dass das vorgesehene Mischungsverhältnis 1:10 oder größer, vorzugsweise 1:4 oder größer, weiter vorzugsweise 1:2 oder größer, besonders bevorzugt 1:1 beträgt, wobei weiter vorzugsweise die Pasten mehr als 3 Monate, vorzugsweise mehr als 6 Monate, besonders bevorzugt mehr als 24 Monate farb- und/oder lagerstabil sind.

7. Polymerisierbares Dentalmaterial nach einem der vorangehenden Ansprüche, **dadurch gekennzeichnet, dass** das Salz der CH-aciden Verbindung der Komponente 1 ein Salz der α-Benzoyl-proprionitrile, α-Cyan-carbonsäureester und -amide, cyclischer β-Oxonitrile, β-Diketone, cyclischer β-Diketone, cyclischer β-Oxocarbonsäureester, cyclischer β-Oxo-lactone, Malonsäure oder Barbitursäure ist, wobei weiter vorzugsweise das Salz der CH-aciden Verbindung ein Salz ausgewählt aus der Gruppe bestehend aus einwertigen und zweiwertigen Salzen der Alkali- und Erdalkaliionen, vorzugsweise ein Natriumsalz ist.

8. Polymerisierbares Dentalmaterial nach einem der vorangehenden Ansprüche, **dadurch gekennzeichnet, dass** die Säure der Komponente 2 eine anorganische Säure, vorzugsweise Phosphorsäure und/oder Salzsäure ist.

9. Polymerisierbares Dentalmaterial nach einem der vorangehenden Ansprüche, **dadurch gekennzeichnet, dass** die Säure eine organische Säure ist, vorzugsweise dass die organische Säure eine Monocarbonsäure, ausgewählt aus der Gruppe bestehend aus Ameisensäure, Essigsäure und Benzoesäure oder eine Dicarbonsäure, ausgewählt aus der Gruppe bestehend aus Oxalsäure, Malonsäure, Succinsäure, Adipinsäure, Pimelinsäure, Azelainsäure, Sebacinsäure, Maleinsäure, Fumarsäure, Sorbinsäure, Phthalsäure und Terephthalsäure oder eine Tricarbonsäure, ausgewählt aus der Gruppe bestehend aus Hemimellithsäure, Trimellithsäure, Trimesinsäure, Agaricinsäure, Citronensäure, 1,2,3-Propantricarbonsäure oder eine Multicarbonsäure, ausgewählt aus der Gruppe bestehend aus Pyromellithsäure und Mellithsäure oder eine Polycarbonsäure, ausgewählt aus der Gruppe bestehend aus Polyacrylsäure und Polymethacrylsäure ist.

10. Polymerisierbares Dentalmaterial nach einem der vorangehenden Ansprüche, **dadurch gekennzeichnet, dass** mindestens eine der Komponenten des polymerisierbaren Dentalmaterials Übergangsmetallkationen, vorzugsweise Cu²⁺-Ionen, und zur Radikalbildung geeignete Anionen, vorzugsweise Halogenid-Ionen, weiter vorzugsweise Chlorid-Ionen, enthält weiter vorzugsweise dass die Komponente 1 Übergangsmetallkationen, vorzugsweise Cu²⁺-Ionen, und zur Radikalbildung geeignete Anionen, vorzugsweise Halogenid-Ionen, weiter vorzugsweise Chlorid-Ionen enthält.

11. Polymerisierbares Dentalmaterial nach einem der vorangehenden Ansprüche, **dadurch gekennzeichnet, dass** mindestens eine der Komponenten des polymerisierbaren Dentalmaterials ein Füllstoff ist, ausgewählt aus der Gruppe bestehend aus micro- und/oder nanoskaligen Füllstoffen, vorzugsweise Metall-, Halbmetall- oder Mischmetalloxide, Silikaten, Nitriden, Sulfaten, Titanaten, Zirkonaten, Stannaten, Wolframaten, Siliciumdioxiden oder einer Mischung aus diesen Verbindungen oder sphärischen Füllstoffen, Quarzpulvern, Glaspulvern, Glaskeramikpulvern oder eine Mischung aus diesen Pulvern oder gefüllten oder ungefüllten Splitterpolymerisaten und/oder Perlpolymerisaten.

12. Polymerisierbares Dentalmaterial nach Anspruch 11, **dadurch gekennzeichnet, dass** der Füllstoff ein oberflächenmodifizierter Füllstoff, vorzugsweise eine organisch oberflächenmodifizierter Füllstoff ist.

13. Polymerisierbares Dentalmaterial nach Anspruch 12, **dadurch gekennzeichnet, dass** der oberflächenmodifizierte Füllstoff auf seiner Oberfläche funktionelle Gruppen besitzt, die mit den Monomeren chemisch, vorzugsweise radikalisch, reagieren können oder eine hohe Affinität zu der aus den Monomeren gebildeten Polymermatrix haben, wobei der Füllstoff vorzugsweise mit reaktiven Acrylat- oder Methacrylatgruppen tragendem Silan oberflächenmodifiziert ist.

14. Verwendung des polymerisierbaren Dentalmaterials nach einem der vorangehenden Ansprüche als Füllungsmaterial, Stumpfaufbaumaterial, Befestigungsmaterial, Bondingmaterial, provisorisches und permanentes Kronen- und Brückenmaterial, zahntechnischen Werkstoff zur Herstellung von Inlays, Onlays, Verblendschalen, künstlichen Zähnen, Modellmaterialien und Fissuren- bzw. Wurzelkanal-Versiegelungsmaterial.

15. Gehärtetes Dentalmaterial, erhältlich aus einem polymerisierbaren Dentalmaterial nach einem der Ansprüche 1 bis 13.

## Claims

1. A polymerisable dental material comprising at least two components, **characterised in that** it comprises the following components:
component 1 containing
a) the salt of a CH-acid compound, wherein the CH-acid compound can initiate a radical polymerisation,
component 2 containing
b) an acid, the acid strength of which is greater than that of the CH-acid compound present as salt in component 1,
wherein the component 1 and the component 2 comprise monomers which can polymerise under radical conditions.

2. A polymerisable dental material according to Claim 1, **characterised in that** polymerisable dental material comprises a total of more than 50% by weight, preferably more than 60% by weight, more preferably more than 70% by weight, more preferably more than 80% by weight, more preferably more than 90% by weight, more preferably more than 95% by weight, more preferably more than 98% by weight, of monomers which can polymerise under radical conditions.

3. A polymerisable dental material according to Claim 1 or 2, **characterised in that** the component 1 comprises more than 50% by weight, preferably more than 60% by weight, more preferably more than 70% by weight, more preferably more than 80% by weight, more preferably more than 90% by weight, more preferably more than 95% by weight, more preferably more than 98% by weight, of monomers which can polymerise under radical conditions.

4. A polymerisable dental material according to any one of the preceding Claims, **characterised in that** the component 1 and/or the component 2 or the polymerisable dental material in total does not comprise any monomer which cannot polymerise under radical conditions.

5. A polymerisable dental material according to any one of the preceding Claims, **characterised in that** the monomers in component 1 can polymerise under radical conditions with the monomers in component 2 when the two components are mixed, and more preferably **in that** the radically polymerisable components are selected from the group comprising acrylic acid ester and methacrylic acid ester.

6. A polymerisable dental material according to any one of the preceding Claims, **characterised in that** the component 1 is contained in a first paste and the component 2 is contained in a second paste, and **in that** the designated mixing ratio is 1:10 or higher, preferably 1:4 or higher, more preferably 1:2 or higher, especially preferably 1:1, wherein more preferably the pastes are stable with regard to colour and/or storage for more than 3 months, preferably more than 6 months, especially preferably more than 24 months.

7. A polymerisable dental material according to any one of the preceding Claims, **characterised in that** the salt of the CH-acid compound of the component 1 is a salt of -benzoyl-propionitriles, .-cyano-carboxylic acid esters and amides, cyclic .-oxonitriles, .-diketones, cyclic .-diketones, cyclic .-carboxylic acid esters, cyclic .-oxo-lactones, malonic acid, malonic acid or barbituric acid, wherein more preferably the salt of the CH-acid compound is a salt selected from the group comprising monovalent and divalent salts of alkali and alkaline earth ions, preferably a sodium salt.

8. A polymerisable dental material according to any one of the preceding Claims, **characterised in that** the acid of the component 2 is an inorganic acid, preferably phosphoric acid and/or hydrochloric acid.

9. A polymerisable dental material according to any one of the preceding Claims, **characterised in that** the acid is an organic acid, preferably **in that** the organic acid is a monocarboxylic acid selected from the group comprising formic oxalic acid, acetic acid and benzoic acid or a dicarboxylic acid selected from the group comprising oxalic acid, malonic acid, succinic acid, adipic acid, pimelic acid, azelaic acid, sebacic acid, maleic acid, fumaric acid, sorbic acid, phthalic acid and terephthalic acid or a tricarboxylic acid selected from the group comprising hemimellitic acid, trimellitic acid, trimesic acid, agaric acid, citric acid, 1,2,3-propanetricarboxylic acid or a multicarboxylic acid selected from the group comprising pyromellitic acid and mellitic acid or a polycarboxylic acid selected from the group comprising polyacrylic acid and polymethacrylic acid.

10. A polymerisable dental material according to any one of the preceding Claims, **characterised in that** at least one of the components of the polymerisable dental material comprises transition metal cations, preferably Cu²⁺ ions, and anions suitable for radical formation, preferably halide ions, more preferably chloride ions, more preferably **in that** the component 1 comprises transition metal cations, preferably Cu²⁺ ions, and anions suitable for radical formation, preferably halide ions, more preferably chloride ions.

11. A polymerisable dental material according to any one of the preceding Claims, **characterised in that** at least one of the components of the polymerisable dental material is a filling material selected from the group comprising micro and/or nanoscale filling materials, preferably metal, semi-metal or mixed metal oxides, silicates, nitrides, sulphates, titanates, zirconates, stannates, tungstates, silicon dioxides or a mixture of these compounds or spherical filling materials, quartz powders, glass powders, glass ceramic powders or a mixture of these powders or filled or unfilled splinter polymers and/or bead polymers.

12. A polymerisable dental material according to Claim 11, **characterised in that** the filling material is a surface-modified filling material, preferably an organically surface-modified filling material.

13. A polymerisable dental material according to Claim 12, **characterised in that** the surface-modified filling material has, on its surface, functional groups which can react chemically, preferably under radical conditions, with the monomers or have a high affinity for the polymer matrix formed from the monomers, wherein the filling material is preferably surface-modified with a silane carrying reactive acrylate or methacrylate groups.

14. Use of a polymerisable dental material according to any one of the preceding Claims as filling material, stump build-up material, fixing material, bonding material, material for temporary and permanent crown and bridge material, material for dental technology for the preparation of inlays, onlays, veneers, artificial teeth, cast materials, fissure-sealing material and root-canal sealing material.

15. A cured dental material, which can be obtained from a polymerisable dental material according to any one of Claims 1 to 13.

## Revendications

1. Matériau dentaire polymérisable en au moins deux composants, **caractérisé en ce qu'**il comprend les composants suivants:
composant 1, contenant
a) le sel d'un composé à CH acide, le composé à CH acide pouvant déclencher une polymérisation radicalaire,
composant 2, contenant
b) un acide dont la l'acidité est plus grande que celle du CH acide présent comme sel dans le composant 1,
sachant que le composant 1 et le composant 2 contiennent des monomères polymérisables par polymérisation radicalaire.

2. Matériau dentaire polymérisable selon la revendication 1, **caractérisé en ce que** le matériau dentaire polymérisable contient en tout plus de 50 % en poids, de préférence plus de 60 % en poids, encore plus préférablement plus de 70 % en poids, encore plus préférablement plus de 80 % en poids, encore plus préférablement plus de 90 % en poids, encore plus préférablement plus de 95 % en poids, encore plus préférablement plus de 98 % en poids de monomères polymérisables par polymérisation radicalaire.

3. Matériau dentaire polymérisable selon la revendication 1 ou 2, **caractérisé en ce que** le composant 1 contient plus de 50 % en poids, de préférence plus de 60 % en poids, encore plus préférablement plus de 70 % en poids, encore plus préférablement plus de 80 % en poids, encore plus préférablement plus de 90 % en poids, encore plus préférablement plus de 95 % en poids, encore plus préférablement plus de 98 % en poids de monomères polymérisables par polymérisation radicalaire.

4. Matériau dentaire polymérisable selon l'une quelconque des revendications précédentes, **caractérisé en ce que** le composant 1 et/ou le composant 2 ou le matériau dentaire polymérisable dans son ensemble ne contiennent pas de monomères non polymérisables par polymérisation radicalaire.

5. Matériau dentaire polymérisable selon l'une quelconque des revendications précédentes, **caractérisé en ce que** les monomères dans le composant 1 peuvent polymériser radicalairement avec les monomères dans le composant 2 lors du mélangeage des deux composants et en outre de préférence **en ce que** les monomères polymérisables par polymérisation radicalaire sont choisis parmi le groupe consistant en les esters de l'acide acrylique et les esters de l'acide méthacrylique.

6. Matériau dentaire polymérisable selon l'une quelconque des revendications précédentes, **caractérisé en ce que** le composant 1 est contenu dans une première pâte et le composant 2 dans une deuxième pâte, et **en ce que** le rapport de mélange prévu est de 1 : 10 ou plus, de préférence de 1 : 4 ou plus, encore plus préférablement de 1 : 2 ou plus, de façon particulièrement préférée de 1 : 1, sachant qu'en outre, les pâtes sont stables à la couleur et/ou au stockage plus de 3 mois, de préférence plus de 6 mois, de façon particulièrement préférée plus de 24 mois.

7. Matériau dentaire polymérisable selon l'une quelconque des revendications précédentes, **caractérisé en ce que** le sel du composé à CH acide du composant 1 est un sel du <-benzoyl-propionitrile, d'un ester ou d'un amide d'un acide <-cyano-carboxylique, d'un β-oxonitrile cyclique, d'une β-dicétone, d'une β-dicétone cyclique, d'un ester d'acide β-oxocarboxylique cyclique, d'une β-oxolactone cyclique, de l'acide malonique ou de l'acide barbiturique, le sel du composé à CH acide étant en outre de préférence un sel choisi parmi le groupe consistant en des sels monovalents et divalents des ions alcalins et alcalinoterreux, de préférence un sel de sodium.

8. Matériau dentaire polymérisable selon l'une quelconque des revendications précédentes, **caractérisé en ce que** l'acide du composant 2 est un acide inorganique, de préférence de l'acide phosphorique et/ou de l'acide chlorhydrique.

9. Matériau dentaire polymérisable selon l'une quelconque des revendications précédentes, **caractérisé en ce que** l'acide est un acide organique, de préférence l'acide organique est un acide monocarboxylique, choisi parmi le groupe consistant en l'acide formique, l'acide acétique et l'acide benzoïque, ou un acide dicarboxylique choisi parmi le groupe consistant en l'acide oxalique, l'acide malonique, l'acide succinique, l'acide adipique, l'acide pimélique, l'acide azélaïque, l'acide sébacique, l'acide maléique, l'acide fumarique, l'acide sorbique, l'acide phtalique et l'acide téréphtalique, ou un acide tricarboxylique, choisi parmi le groupe consistant en l'acide hémimellitique, l'acide trimellitique, l'acide trimésique, l'acide agaricique, l'acide citrique, l'acide propane 1,2,3-tricarboxylique, ou un acide multicarboxylique, choisi parmi le groupe consistant en l'acide pyromellitique et l'acide mellitique, ou un acide polycarboxylique, choisi parmi le groupe consistant en l'acide polyacrylique et l'acide polyméthacrylique.

10. Matériau dentaire polymérisable selon l'une quelconque des revendications précédentes, **caractérisé en ce qu'**au moins un des composants du matériau dentaire polymérisable contient des cations de métaux de transition, de préférence des ions Cu²⁺, et des anions appropriés à la formation de radicaux, de préférence des ions halogénures, plus préférablement des ions chlorures, le composant 1 contenant de préférence des cations de métaux de transition, de préférence des ions Cu²⁺, et des anions appropriés à la formation de radicaux, de préférence des ions halogénures, plus préférablement des ions chlorures.

11. Matériau dentaire polymérisable selon l'une quelconque des revendications précédentes, **caractérisé en ce qu'**au moins un des composants du matériau dentaire polymérisable est une charge, choisie parmi le groupe consistant en des charges à l'échelle micrométrique et/ou nanométrique, de préférence des oxydes métalliques, semi-métalliques, ou métalliques mixtes, des silicates, des nitrures, des sulfates, des titanates, des zirconates, des stannates, des tungstates, des dioxydes de silicium ou un mélange de ces composés, ou des charges sphériques, des poudres de quartz, des poudres de verre, des poudres de céramique de verre ou un mélange de ces poudres ou des polymères en copeaux et/ou des polymères perlés chargés ou non chargés.

12. Matériau dentaire polymérisable selon la revendication 11, **caractérisé en ce que** la charge est une charge modifiée en surface, de préférence une charge modifiée en surface organiquement.

13. Matériau dentaire polymérisable selon la revendication 12, **caractérisé en ce que** la charge modifiée en surface possède sur sa surface des groupes fonctionnels qui peuvent réagir chimiquement avec les monomères, de préférence de façon radicalaire, ou qui ont une haute affinité pour la matrice de polymère formée à partir des monomères, la charge étant de préférence modifiée en surface avec un silane portant des groupes réactifs acrylates ou méthacrylates.

14. Utilisation du matériau dentaire polymérisable selon l'une quelconque des revendications précédentes comme matériau d'obturation, matériau de construction de moignon, matériau de fixation, matériau de collage, matériau pour des couronnes et des bridges provisoires ou permanents, matériau dentaire technique pour la fabrication d'inlays, d'onlays, de coques de revêtement, de dents artificielles, de matériaux pour modèles et de matériaux pour le scellement de fissures et de canaux radiculaires.

15. Matériau dentaire durci pouvant être obtenu à partir d'un matériau dentaire polymérisable selon l'une quelconque des revendications 1 à 13.
